# EUROPEAN PATENT APPLICATION

(11) **EP 4 556 910 A1**
(43) Date of publication of application: **21.05.2025**
(21) Application number: 23759244.9
(22) Date of filing: 23.02.2023
(51) Int. Cl.: G01N 35/00

(54) **MOLECULAR DIAGNOSIS APPARATUS-BASED CONTROL METHOD AND MOLECULAR DIAGNOSIS APPARATUS**

(30) Priority: 25.02.2022 CN 202210178016
(71) Applicant: Edan Instruments, Inc., Shenzhen, Guangdong 518000 (CN)
(72) Inventor: PI, Shiwei, Shenzhen, Guangdong 518000 (CN); CHEN, Jiaqi, Shenzhen, Guangdong 518000 (CN); ZHANG, Xing, Shenzhen, Guangdong 518000 (CN)
(74) Representative: Herrero & Asociados, S.L.
(86) International application number: PCT/CN2023/077940
(87) International publication number: WO 2023/160622

(57) **Abstract**

A molecular diagnosis apparatus (100)-based control method and a molecular diagnosis apparatus (100), relating to the technical field of molecular testing. The control method comprises: controlling preheating of a first heating element (55, 832) corresponding to a sample feeding cavity (9411) on a test card (93); controlling the first heating element (55, 832) to heat the sample feeding cavity (9411); controlling preheating of a second heating element (54, 8211) corresponding to a test cavity (9461) on the test card (93); and controlling the second heating element (54, 8211) to heat the test cavity (9461). During the heating process, by separately heating the sample feeding cavity (9411) and the test cavity (9461) of the test card (93), the effect of saving resources is achieved; in addition, during the heating process, preheating is separately performed on the first heating element (55, 832) for heating the sample feeding cavity (9411) and the second heating element (54, 8211) for heating the test cavity (9461), so that the heating process and testing processes such as a test card (93) placement process and a centrifugal treatment process can be parallel during the test process of the molecular diagnosis apparatus (100), thus shortening time for heating, and improving the test efficiency of the test card (93).

## Description

### CROSS REFERENCE TO RELATED APPLICATIONS

This application claims priority to Chinese Patent Application No. 202210178016.6, filed on February 25, 2022 and entitled "molecular diagnostic device and control method based on molecular diagnostic device".

### TECHNICAL FIELD

The present disclosure relates to the field of molecular detection technologies, specifically to a molecular diagnostic device and a control method based on the molecular diagnostic device.

### BACKGROUND

Molecular diagnostic devices utilize molecular diagnostic technologies. The molecular diagnostic technologies refer to diagnostic techniques that use nucleic acids or proteins as biomarkers for clinical testing, and may provide information and decision-making support for disease prediction, diagnosis, prevention, treatment, and prognosis.

When molecular diagnostic device performs testing on a test card, the test card requires to be heated. However, when existing heating devices are applied to multiple channels, resource wastage may easily occur.

### SUMMARY

According to a first aspect, a control method based on a molecular diagnostic device is provided. The control method may include: controlling a first heating component for a sample loading cavity of a test card to perform preheating; controlling the first heating component to heat the sample loading cavity; controlling a second heating component for a testing cavity of the test card to perform preheating; and, controlling the second heating component to heat the testing cavity.

According to another aspect, a molecular diagnostic device is further provided in the present disclosure. The molecular diagnostic device includes a first heating component, a second heating component and a control unit. The first heating component is configured to be arranged matching a sample loading cavity of a test card. The second heating component is configured to be arranged matching a testing cavity of the test card. The control unit is connected to the first heating component and the second heating component respectively, configured to control the first heating component to perform preheating, heat the sample loading cavity after preheating, and then control the second heating component to perform preheating and heat the testing cavity after preheating.

### BRIEF DESCRIPTION OF THE DRAWINGS

To more clearly illustrate technical schemes in the present disclosure, the drawings required in the description of the embodiments will be briefly introduced below. Obviously, the drawings in the following description are only some embodiments of the present disclosure. For those of ordinary skills in the art, other drawings could be obtained based on these drawings without creative efforts.
Fig.1 is a schematic diagram of a three-dimensional structure of a molecular diagnostic device according to an embodiment of the present disclosure.
Fig.2 is an exploded schematic view of the molecular diagnostic device in Fig.1.
Fig. 3 is an exploded schematic view of a pressure-bearing disc in Fig. 2.
Fig. 4 is a schematic structural diagram of a first housing in Fig. 3.
Fig. 5 is a schematic structural diagram of the pressure-bearing disc in Fig.3.
Fig. 6 is a schematic structural diagram of a delivery assembly in Fig.2.
Fig. 7 is a schematic structural diagram of a deliver component in Fig. 6.
Fig. 8 and Fig. 9 are schematic structural diagrams from different viewing angles, illustrating the cooperation between the pressure-bearing disc and the delivery assembly in Fig. 2.
Fig. 10 is an exploded view of a test card testing base in Fig 2.
Fig.11 is an exploded view of a support base in Fig.10.
Fig.12 is a schematic structural diagram of a support base main body in Fig. 11.
Fig. 13 is a schematic structural diagram of a light test component in Fig. 11.
Fig. 14 is a schematic structural diagram of a sample loading cavity assembly in Fig. 11.
Fig. 15 is a schematic structural diagram of a light generator in Fig. 10.
Fig. 16 is a schematic structural diagram of a test card according to one embodiment of the present disclosure.
Fig. 17 is a cross-sectional schematic view of the test card along line L-L in Fig. 16.
Fig. 18 is a schematic three-dimensional structural diagram of the test card in Fig. 16.
Fig. 19 is a schematic flowchart of a usage process of the test card in Fig. 18.
Fig. 20 is a schematic flowchart of a control method based on a molecular diagnostic device according to an embodiment of the present disclosure.
Fig. 21 is a structural schematic diagram of a molecular diagnostic device according to one embodiment of the present disclosure.

### DETAILED DESCRIPTION

Technical schemes in embodiments of the present disclosure will be described clearly and thoroughly in connection with accompanying drawing of the embodiments of the present disclosure. Obviously, the described embodiments are only a part of the embodiments, but not all of them. All other embodiments by a person of ordinary skills in the art based on embodiments of the present disclosure without creative efforts should all be within the protection scope of the present disclosure.

Reference to "embodiments" herein means that a specific feature, structure, or characteristic described in conjunction with the embodiments may be included in at least one embodiment of the present disclosure. The appearance of this phrase in various locations in the specification does not necessarily refer to the same embodiment, nor is it an independent or alternative embodiment mutually exclusive with other embodiments. Those skilled in the art may explicitly and implicitly understand that, the embodiments described herein may be combined with other embodiments.

A molecular diagnostic device, which utilizes molecular diagnostic technologies, will be described hereinafter. Molecular diagnostic technology refers to diagnostic techniques that use nucleic acids or proteins as biomarkers for clinical testing, and may provide information and decision-making support for disease prediction, diagnosis, prevention, treatment, and prognosis. Especially in the face of various emerging infectious diseases, the most cost-effective and efficient measure is rapid and accurate molecular diagnostics.

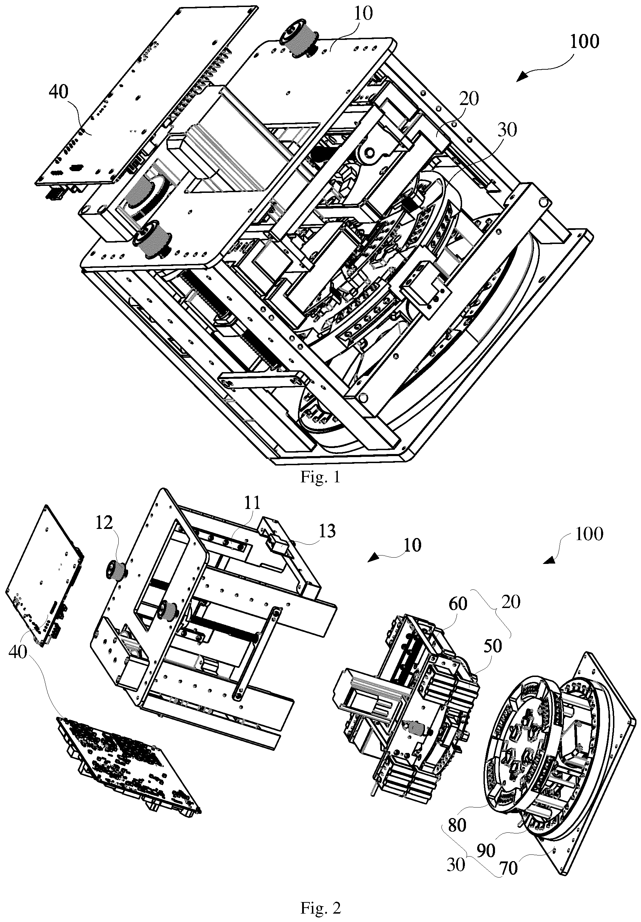

As illustrated in Fig. 1 and Fig. 2, Fig.1 is a schematic diagram of a three-dimensional structure of a molecular diagnostic device 100 according to an embodiment of the present disclosure. Fig.2 is an exploded view of the molecular diagnostic device 100 in Fig.1. The molecular diagnostic device 100 may include a frame 10, a test card conveyor base 20 mounted on the frame 10, a test card testing base 30 mounted to the frame 10, and a control circuit board 40 mounted to the frame 10.

The test card conveyor base 20 may be configured to hold a test card. The test card conveyor base 20 may slide relative to the frame 10, such that the test card conveyor base 20 may carry the test card, and deliver the test card to the test card testing base 30. The test card testing base 30 may be configured to generate excitation light, so as to test the test card and produce a test signal. The control circuit board 40 may control the sliding of the test card conveyor base 20 on the frame 10, control the test card testing base 30 to perform test on the test card, and receive and process the test signal to generate diagnostic data.

In some embodiments, the molecular diagnostic device 100 may further include input devices, such as a display, a keyboard, and a code scanning device 13 (as illustrated in Fig. 2), which may be electrically connected to the control circuit board 40. Controlling instructions may be allowed to be input, through these input devices, to the molecular diagnostic device 100, such as to the control circuit board 40, thereby enabling the molecular diagnostic device 100 to control the test card conveyor base 20 and/or the test card testing base 30 via the control circuit board 40.

In the present disclosure, the terms ""up", "down", "front", "back", "left", "right", "top", "bottom", "upper" and "lower" may be used for description purposes. In the present disclosure, the terms "middle", "longitudinal", "transverse", "length", "width", "thickness", "upper", "lower", "front", "rear", "left", "right", "vertical", "horizontal", "top", "bottom", "inner", "outer", "clockwise", "counter-clockwise" and others may indicate directions or positions that are based on the orientation or position relationship shown in the drawings, and are only for the convenience of describing the application and simplification of the description, but not indicate or imply that the apparatus or unit referred to must have a specific orientation, be constructed and operated in a specific orientation, therefore cannot be construed as a restriction on this application.

In addition, the terms "first" and "second" etc. in the present specification are only for the purpose of description and cannot be construed as indicating or implying relative importance or implicitly indicating the number of technical features referred to. Thus, the features preceded by "first" and "second" may explicitly or implicitly may include one or more of these features.

As illustrated in Fig. 2, the test card conveyor base 20 may include a pressure-bearing disc 50 and a delivery assembly 60. The pressure-bearing disc 50 is mounted on the frame 10, such as on a first guide rail 11. The delivery assembly 60 is configured to hold the test card. The pressure-bearing disc 50 is threadedly connected to the frame 10, such as to a lead screw 12. The pressure-bearing disc 50 may slide on the frame 10, for example, along the first guide rail 11, thereby causing the delivery assembly 60 to slide on the frame 10, for example along the first guide rail 11. The delivery assembly 60 may carry the test card, and deliver the test card to the test card testing base 30.

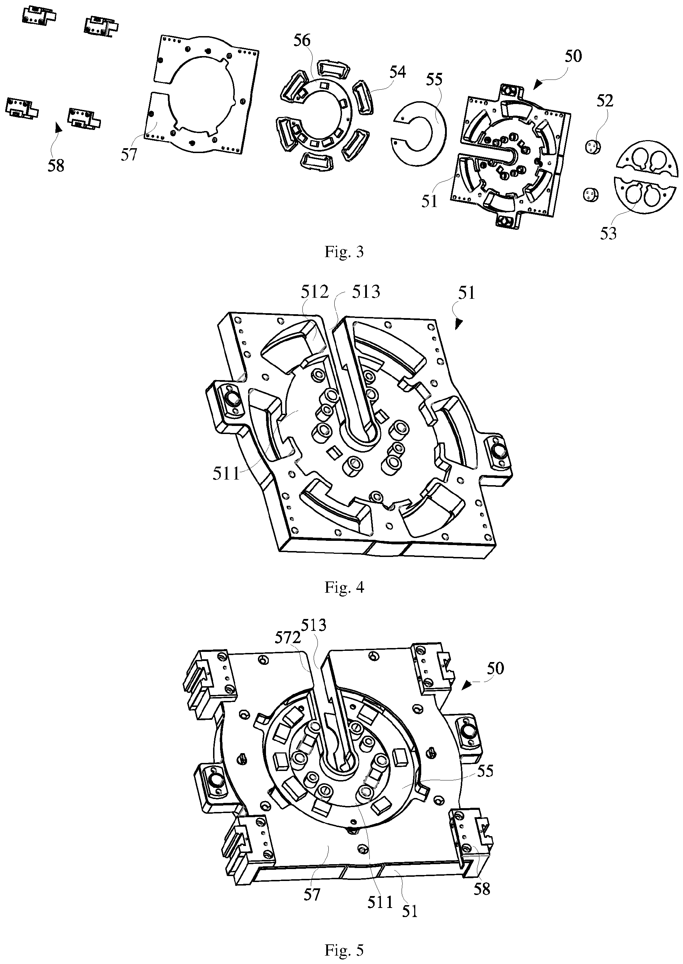

As illustrated in Fig. 3, Fig. 3 is an exploded view of the pressure-bearing disc 50 in Fig. 2. The pressure-bearing disc 50 may include a first housing 51, an electromagnetic component 52, a tight-pressing member 53, a first heating component 54, a second heating component 55, a first circuit board 56, a second housing 57, and a first sliding rail 58. The first housing 51 is mounted on the frame 10, for example on the lead screw 12. The electromagnetic component 52 is arranged on a side of the first housing 51 facing the test card testing base 30. The tight-pressing member 53 is configured to secure the test card on the delivery assembly 60. The first heating component 54 and the second heating component 55 are arranged on the first housing 51. The first circuit board 56 is mounted on the first housing 51, and is electrically connected to the electromagnetic component 52, the first heating component 54, and the second heating component 55 respectively. The second housing 57 is configured to cover a side of the first housing 51 away from the electromagnetic component 52. The first sliding rail 58 is arranged on the first housing 51, and is mounted on the frame 10, for example on the first guide rail 11 of the frame 10.

The first housing 51 and the second housing 57 are interlocked or engaged to form a main body of the pressure-bearing disc. The first circuit board 56 may be electrically connected to the control circuit board 40. The electromagnetic component 52 may generate a magnetic force under control of the control circuit board 40, so as to attract the tight-pressing member 53 onto the first housing 51. The electromagnetic component 52 may also eliminate the magnetic force under the control of the control circuit board 40, so as to avoid attracting the tight-pressing member 53. The first heating component 54 and the second heating component 55 may heat the test card under the control of the control circuit board 40. The first sliding rail 58 may slide on the frame 10, for example along the first guide rail 11. The first housing 51 is connected to the frame 10, for example to the lead screw 12, so as to simultaneously slide along the extension direction of both the lead screw 12 and the first guide rail 11.

As illustrated in Fig. 4, Fig. 4 is a schematic structural diagram of the first housing 51 in Fig.3. A receiving slot 511 is defined in the center of a side of the first housing 51 facing the second housing 57, and is configured to receive the first circuit board 56. The cross section of the receiving slot 511 may be circular, or may be of other shapes.

Snap-fit holes 512 may be defined in the first housing 51 around the receiving slot 511. The snap-fit holes 512 may be configured to install the first heating component 54. The snap-fit holes 512 may be circumferentially and evenly distributed around the receiving slot 511. The number of the snap-fit holes 512 may be 1 or more. In some embodiments, the number of the snap-fit holes 512 may also be one of 2, 3, 4, 5, 6, etc. In some embodiments, the number of the snap-fit holes 512 may be 6. In some embodiments, the shape of a snap-fit hole 512 is a segment of a ring structure.

The first housing 51 defines a notch 513. The notch 513 is configured to allow the passage of the delivery assembly 60 or provide space for the delivery assembly 66. The notch 513 extends inward from an edge of the first housing 51 near the code scanning device 13 toward the interior of the first housing 51. In some embodiments, the notch 513 is defined between two adjacent snap-fit holes 512.

As further illustrated in Fig. 3, the electromagnetic component 52 may be powered to generate the magnetic force, thereby attracting and securing the tight-pressing member 53. The electromagnetic components 52 are electrically connected to the first circuit board 56. The number of the electromagnetic components 52 may be two. The electromagnetic components 52 may be mounted on the side of the first housing 51 away from the second housing 57.

As further illustrated in Fig. 3, the tight-pressing member 53 may be made of rigid materials such as metal. In some embodiments, the tight-pressing member 53 may be made of a metal (such as iron), which may be attracted by the magnetic force of an electromagnet. The tight-pressing member 53 is arranged on a side of the first housing 51 away from the second housing 57. When the electromagnetic component 52 attracts the tight-pressing member 53, it can attract the tight-pressing member 53.

As further illustrated in Fig. 3, the first heating component 54 is mounted on a side of the first housing 51 close to the second housing 57. Heating devices such as heating resistors etc. may be arranged inside the first heating component 54. The number of the first heating components 54 may be multiple. In some embodiments, the number of the first heating components 54 may be one of 2, 3, 4, 5, 6, etc. In some embodiments, the number of first heating components 54 may be consistent with the number of the snap-fit holes 512, which may be 6.

The first heating components 54 may be arranged within the first housing 51, for example, in the snap-fit holes 512, and are snap-fitted to the first housing 51, such that the first heating components 54 are mounted on the first housing 51.

In some embodiments, the first circuit board 56 is electrically connected to the first heating component 54, so as to control the heating devices inside the first heating component 54.

As further illustrated in Fig. 3, the second heating component 55 is mounted on a side of the first housing 51 away from the second housing 57. The second heating component 55 may as a whole have a ring-shaped structure. The second heating component 55 may be made of thermally conductive rigid materials, such as metal. Heating devices, such as heating resistors etc. may be arranged inside the second heating component 55. In some embodiments, the first circuit board 56 is electrically connected to the heating devices inside the second heating component 55, so as to control the heating devices inside the second heating component 55.

As further illustrated in Fig. 3, electronic components, such as resistors, capacitors, and inductors are arranged on the first circuit board 56. The first circuit board 56 is mounted within an accommodating groove 511 of the first housing 51. The first circuit board 56 may be electrically connected to the heating devices inside the first heating component 54, the heating devices inside the second heating component 55 and the electromagnetic component 52 respectively, so as to control the heating devices inside the first heating component 54, the heating devices inside the second heating component 55 and the electromagnetic component 52 respectively.

As illustrated in Fig. 5, Fig.5 is a schematic structural diagram of the pressure-bearing disc 50 in Fig. 3. The second housing 57 may be fixedly connected to the first housing 51 through connection manners such as screw connection, a plug connection, a snap-fit connection, welding, or bonding etc., which will not be detailed here. The second housing 57 fully covers the first housing 51.

A notch 572 is defined in the second housing 57. The notch 572 is defined on the second housing 57 at a location matching or aligned with the notch 513, so that when the second housing 57 is engaged with the first housing 51, the notch 572 communicates with the notch 513, to provide a space for the delivery assembly 60, thereby allowing the delivery assembly 60 to slide within the notch 572 and the notch 513.

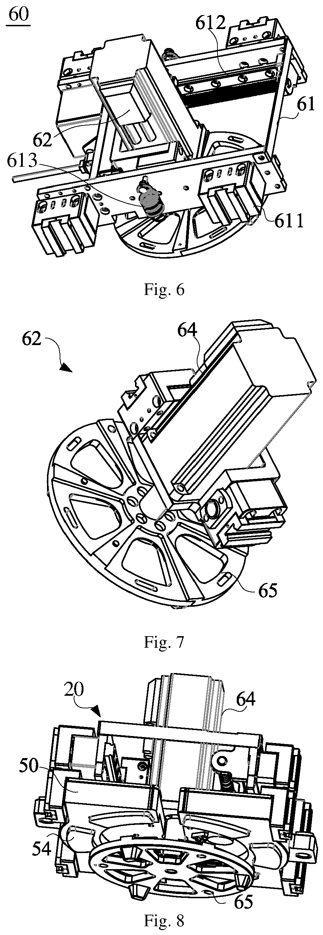

As illustrated in Fig. 6, Fig.6 is a schematic structural diagram of the delivery assembly 60 in Fig. 2. The delivery assembly 60 may include a sliding carriage 61 and a delivery component 62 mounted on the sliding carriage 61. The sliding carriage 61 is positioned above the pressure-bearing disc 50 and mounted on the frame 10, for example, on the first guide rail 11 of the frame 10. The sliding carriage 61 may slide on the frame 10, for example along the first guide rail 11. The sliding carriage 61 is connected to the pressure-bearing disc 50, allowing them to slide together on the frame 10 in certain scenarios. The delivery component 62 may be configured to hold the test card. The delivery component 62 may slide relative to the sliding carriage 61. A sliding direction of the delivery component 62 relative to the sliding carriage 61 is different from the sliding direction of the sliding carriage 61 relative to the frame 10.

When the delivery assembly 60 extends, the delivery component 62 slides on the sliding carriage 61 to a first position outside the frame 10, completing the extension of the delivery assembly 60. The test card is placed while the delivery assembly 60 is in an extended state. Then, when the delivery assembly 60 contracts, the delivery component 62 slides on the sliding carriage 61 to a second position within the frame 10, completing the contraction of the delivery assembly 60. When the delivery assembly 60 is in the contracted state, it may slide between a third position and a fourth position on the first guide rail 11. In some embodiments, the delivery assembly 60 may perform extension when in the third position. In some embodiments, the delivery assembly 60 may perform extension at a position between the third position and the fourth position.

The sliding carriage 61 is provided with a second sliding rail 611. The second sliding rail 611 is slidably connected to the frame 10, such as to the first guide rail 11.

The sliding carriage 61 is provided with a second guide rail 612. The second guide rail 612 is configured for mounting the delivery component 62, thereby allowing the delivery component 62 to slide along the second guide rail 612. The extension direction of the second guide rail 612 may be aligned with or consistent with the extension direction of the notch 513, so that as the delivery component 62 slides along the second guide rail 612, it also slides within the notch 513. In some embodiments, to enable the pressure-bearing disc 50 to slide together with the sliding carriage 61, a traction component 613 may be provided on the sliding carriage 61. The traction component 613 may include a tension spring. For example, one end of the tension spring is connected to the sliding carriage 61, and another end of the tension spring is connected to the second housing 57.

As illustrated in Fig. 7, Fig. 7 is a schematic structural diagram of the delivery component 62 in Fig. 6. The delivery component 62 may include a third driving assembly 64 and a card holder 65. The third driving assembly 64 is mounted on the second guide rail 612. The card holder 65 is mounted on the third driving assembly 64 and may be driven by the third driving assembly 64 to perform rotational motion. The third driving assembly 64 slides on the second guide rail 612 to move the card holder 65. The card holder 65 may be moved to a position outside the frame 10, such that the test card may be placed on the card holder 65. The third driving assembly 64 may also move the card holder 65 to a position inside the frame 10. The third driving assembly 64 may drive the test card on the card holder 65 to perform centrifugal motion, to complete a centrifugal treatment of the test card.

The card holder 65 is placed below the third driving assembly 64 and is fixedly connected to the third driving assembly 64. The card holder 65 is in the form of a rotary body. The axis of the rotary body is coaxially aligned with an output shaft of the third driving assembly 64. In some embodiments, the card holder 65 has a circular disc-shaped structure. A part of the card holder 65 connected to the third driving assembly 64 is configured as a center, the test cards are arranged on the card holder 65 radially outward from the center. The number of the test cards may be the same as the number of the first heating component 54. Of course, the number of the test cards may also be less than the number of the first heating element 54. In some embodiments, the number of the test cards may be 6, although the exact number of the test cards may be adjusted based on actual situations.

The card holder 65 may engage with the tight-pressing member 53 when in contact with the tight-pressing member 53, so that the test cards are clamped between the card holder 65 and the tight-pressing member 53. The engagement mating relationship between the card holder 65 and the tight-pressing member 53 may involve engagement structures such as a groove and protruding column, an insert structure, or a snap-fit structure etc.

When the card holder 65 delivers the test cards to the test card testing base 30, the delivery assembly 62 may be located at the fourth position.

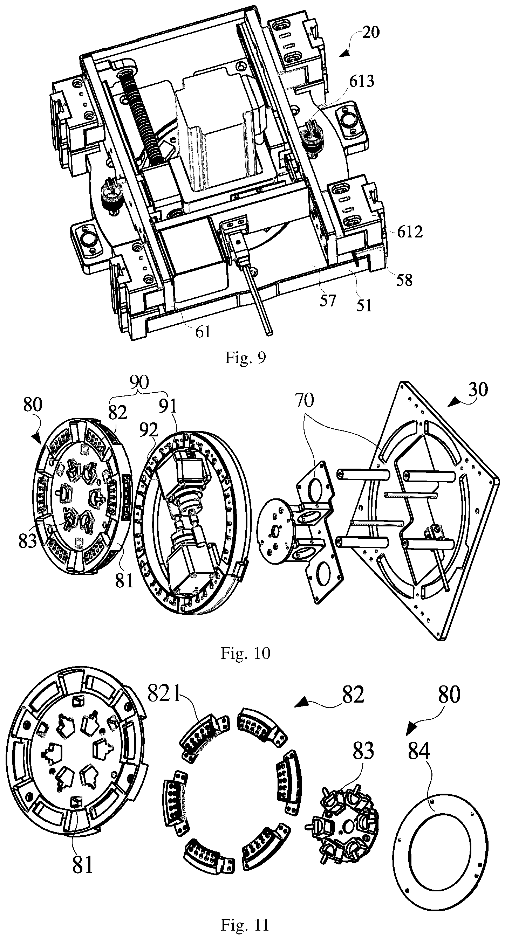

As illustrated in Fig. 8 and Fig. 9, Fig. 8 is a schematic structural diagram from a viewing angle, illustrating the cooperation between the pressure-bearing disc 50 and the delivery assembly 60 in Fig. 2. Fig. 9 is another schematic structural diagram from a viewing angle different from that of Fig. 8, illustrating the cooperation between the pressure-bearing disc and the delivery assembly in Fig. 2. In order to connect the pressure-bearing disc 50 to the delivery assembly 60, the components of the delivery assembly 60, excluding the card holder 65, are first assembled together. Then, the sliding carriage 61 of the delivery assembly 60 is placed above the pressure-bearing disc 50. The second sliding rail 611 of the sliding carriage 61 is arranged opposite to the first sliding rail 58 of the pressure-bearing disc 50, so that the second sliding rail 611 and the first sliding rail 58 are mounted together on the frame 10, for example, on the first guide rail 11 of the frame 10.

Next, the card holder 65 of the delivery component 62 in the delivery assembly 60 is placed on a side of the pressure-bearing disc 50 away from the sliding carriage 61, so that the tight-pressing member 53 is positioned between the first housing 51 and the card holder 65. The output shaft of the third driving assembly 64 of the delivery component 62 in the delivery assembly 60 passes through the notch of the pressure-bearing disc 50 and is fixedly connected to the card holder 65. The second housing 57 and the traction component 613 in the delivery component 60 are arranged opposite to and connected to each other.

As illustrated in Fig. 10, Fig. 10 is an exploded view of the test card testing base 30 in Fig. 2. The test card testing base 30 may include a support frame 70, a support base 80 and a testing assembly 90. The support frame 70 is fixedly connected to the frame 10. The support base 80 is mounted on the support frame 70. The testing assembly 90 is mounted on both the support frame 70 and the support base 80. The support base 80 is positioned below the pressure-bearing disc 50, so as to carry the test cards conveyed by the test card conveyor base 20, such as by the delivery assembly 60. The testing assembly 90 is electrically connected to the control circuit board 40. When the test cards are placed on the support base 80, the testing assembly 90 may perform a series of testing processes on the test card using the excitation light to generate testing signals. The testing assembly 90 then transmits the testing signals to the control circuit board 40.

As illustrated in Fig. 11, Fig.11 is an exploded view of the support base 80 in Fig.10. The support base 80 may include a support base main body 81, a testing cavity assembly 82, a sample loading cavity assembly 83 and a second circuit board 84. The support base main body 81 is mounted on the support frame 70. The testing cavity assembly 82 is mounted on the support base main body 81. The sample loading cavity assembly 83 is mounted on the support base main body 81 and is configured to support the test card in cooperation with the testing cavity assembly 82. The second circuit board 84 is arranged on the support base main body 81. The testing cavity assembly 82 may be mounted on the support base 80 at a position opposite to the first heating component 54, so as to cooperate with the first heating component 54. The sample loading cavity assembly 83 may be mounted on the support base 80 at a position opposite to the second heating component 55, so as to cooperate with the second heating component 55. The testing cavity assembly 82 and the sample loading cavity assembly 83 may cooperate together to support the test card and to perform heating on the test card. The testing cavity assembly 82 may also be configured to perform an excitation light test on the test card. Both the testing cavity assembly 82 and the sample loading cavity assembly 83 are electrically connected to the second circuit board 84, so as to enable heating control of the testing cavity assembly 82 and the sample loading cavity assembly 83.

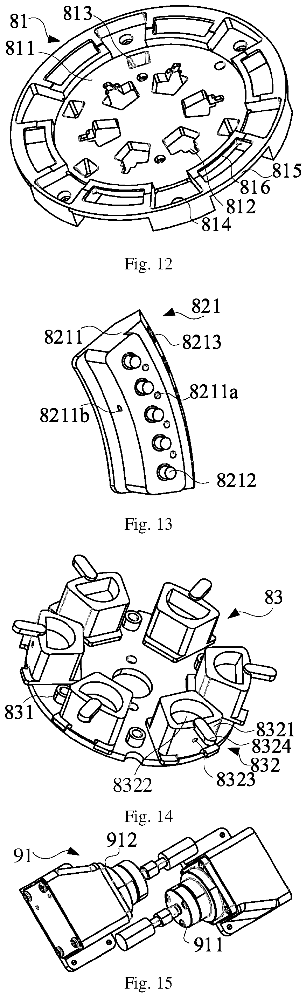

As illustrated in Fig. 12, Fig.12 is a schematic structural diagram of the support base main body 81 in Fig. 11. The support base main body 81 may have a plate-like structure as a whole. A placement slot 811 may be defined on the top of the support base main body 81, so as to provide space for the card holder 65.

A fastening hole 812 is defined in the placement slot 811 of the support base main body 81, so as to cooperate with the sample loading cavity assembly 83. A positioning component 813 is further provided within the placement slot 811 of the support base main body 81, and is configured to cooperate with the card holder 65 to position the test card. In some embodiments, the positioning component 813 may be a positioning slot defined within the placement slot 811 of the support base main body 81, and is configured to position the test card when the card holder 65 (such as a positioning block) is placed into the positioning component 813 (such as a positioning slot). In some embodiments, the shape of the positioning component 813 (such as the positioning block) may match the shape of the card holder 65 (such as the positioning slot). The positioning and mating relationship between the support base main body 81 and the card holder 65 is not limited to the mating relationship between the positioning component 813 and the card holder 65, the relationship may also include magnetic interactions between magnets, magnetic forces between electromagnets, and of course may be other kind of mating relationships, which will not be detailed here.

A plurality of extending slots 814 are circumferentially and evenly distributed around the placement slot 811 of the support base main body 81. The extending slots 814 communicate with the placement slot 811, such that the support base main body 81 may form an assembly platform 815 between two adjacent extending slots 814. The test card may be placed on the assembly platform 815. The extending slots 814 provide clearance or space for the test cards, allowing the assembly platform 815 to support and secure the test card.

A receiving hole 816 is defined by the support base main body 81 at the location of the assembly platform 815, such that the testing cavity assembly 82 is mounted within the receiving hole 816.

As illustrated in Fig. 11, the testing cavity assembly 82 is mounted within the support base main body 81, for example within the receiving hole 816. The testing cavity assembly 82 may include at least one light test component 821. The specific number of the light test components 821 is one of 1, 2, 3, 4, 5, or 6 etc., such that the testing cavity assembly 82 may be mounted in the receiving hole 816.

The light test component 821 may be arranged to match with a first heating component 54, such that the light test component 821 and the first heating component 54 may cooperate to heating the test card. As illustrated in Fig. 13, Fig. 13 is a schematic structural diagram of the light test component 821 in Fig. 11. The light test component 821 may include a testing base 8211, which is positioned within the support base main body 81, for example within the receiving hole 816. The testing base 8211 may be made of a rigid material such as plastic or metal etc. The testing base 8211 is configured to extend toward one side of the receiving hole 816 and project into the receiving hole 816. The testing base 8211 is, on the side of the support base main body 81 where the assembly platform 815 is arranged, flushed with a surface of the assembly platform 815, thereby enhancing an aesthetic appeal of the support base 80, and supporting the test card.

In an extending direction of the testing base 8211, an excitation optical fiber 8212 is provided, such that the excitation optical fiber 8212 may emit light, such as the excitation light, toward the test card. At a position angled relative to the extension direction, a reception optical fiber 8213 is provided for the testing base 8211. The reception optical fiber 8213 is configured to receive both the excitation light emitted from the excitation optical fiber 8212 and fluorescence produced by the excitation light irradiating the test card.

On a side of the testing base 8211 where the excitation optical fiber 8212 is arranged, a heating component receiving slot 8211a is defined. The heating component receiving slot 8211a is configured to house the heating devices such as heating resistors etc. The testing base 8211 may heat the test card by using these heating devices. The heating devices within the heating component receiving slot 8211a may be electrically connected to the second circuit board 84, so as to achieve heating under the control of the second circuit board 84.

On a side of the testing base 8211 approximate to the sample loading cavity assembly 83, a heating component receiving slot 8211b is defined. The heating component receiving slot 8211b is configured to house the heating devices such as heating resistors etc. The testing base 8211 may heat the test card by using these heating devices. The heating devices within the heating component receiving slot 8211b may be electrically connected to the second circuit board 84, so as to achieve heating under the control of the second circuit board 84.

After the heating devices are arranged within the testing base 8211, an effect of heating the test card is provided. Therefore, the testing base 8211 may be referred to as the "third heating component". In some embodiments, the testing base 8211 and the support base main body 81 may be integrated as a single-piece structure.

As illustrated in Fig. 14, Fig. 14 is a schematic structural diagram of the sample loading cavity assembly 83 in Fig. 11. The sample loading cavity assembly 83 may include a support plate 831 and a sample loading cavity mounting seat 832. The support plate 831 is fixed to the support frame 70. The sample loading cavity mounting seat 832 is mounted on the support plate 831. The sample loading cavity mounting seat 832 is configured to secure the test card, and to heat the test card. The sample loading cavity mounting seat 832 is configured to cooperate with the second heating component 55 to heat the test card.

The support plate 831 may be made of a rigid material such as plastic or metal etc. The support plate 831 may have a plate-like structure as a whole, and of course may have other structure, which will not be detailed here. The support plate 831 may be fixedly connected to the support frame 70 through connection manners such as a screw connection, a plug connection, a bonding connection, welding, etc. In some embodiments, the support plate 831 may be fixedly connected to the support frame 70 through connection manners such as a screw connection, a plug connection, a bonding connection, welding, etc. In some embodiments, the support plate 831 may be omitted. In this situation, the sample loading cavity mounting seat 832 may be fixedly connected to the support frame 70 directly. In some embodiments, in case that the support frame 70 is omitted, the support plate 831 may be fixedly connected to the support frame 70.

The sample loading cavity mounting seat 832 may be configured to hold the test card. The sample loading cavity mounting seat 832 may include a mounting seat main body 8321. The mounting seat main body 8321 may as a whole be made of a rigid and thermally conductive material such as plastic or metal etc. The mounting seat main body 8321 is configured to be placed within the support base main body 81, for example, within the fastening hole 812. A sample loading cavity accommodation slot 8322 is defined on a side of the mounting seat main body 8321 away from the support plate 831, and is configured to mount the test card.

A heating component receiving slot 8323 is defined in the mounting seat main body 8321, configured to house the heating devices such as heating resistors etc., such that the mounting seat main body 8321 may heat the test card. The heating devices may be electrically connected to the second circuit board 84, so as to perform heating under the control of the second circuit board 84.

After the heating devices are arranged within the mounting seat main body 8321, the mounting seat main body 8321 have the effect of heating the test card. Therefore, the sample loading cavity mounting seat 832 may be referred to as a "fourth heating component".

The names such as the "first heating element", the "second heating element", the "third heating element", the "fourth heating element", the "fifth heating element", the "sixth heating element," and "heating element," they may be interchangeable in some embodiments. The names such as the "first heating component", the "second heating component", the "third heating component", the "fourth heating component", the "fifth heating component", the "sixth heating component" and "heating component" etc. may be interchanged in some embodiment. For example, in one embodiment, the "first heating component" of other embodiments may be referred to as the "second heating component". Similarly, the "second heating component" of other embodiments may be referred to as the "first heating component".

To better heat the test card, prevent the water vapor condensation from affecting the test process after heating, and enhance the test accuracy, an abutting portion 8324 is provided on a side edge of the sample loading cavity accommodation slot 8322 for the mounting seat main body 8321, and is configured to abut against the test card. In some embodiments, the abutting portion 8324 may further be configured to position the test card.

As illustrated in Fig. 11, the second circuit board 84 may be electrically connected to the heating devices in both the testing cavity assembly 82 and the sample loading cavity assembly 83, so as to control the heating devices for heating.

The second circuit board 84 may have an annular-shaped structure, and may be sleeved around the sample loading cavity assembly 83. The second circuit board 84 may be directly fixed to the side of the support base main body 81 near the support frame 70. In some embodiments, the second circuit board 84 may be directly fixed to the support frame 70. In some embodiments, the second circuit board 84 may be directly fixed to the support frame 70. In some embodiments, the second circuit board 84 may also be directly fixed to the sample loading cavity assembly 83, such as the support plate 831.

In some embodiments, the second circuit board 84 may be omitted while being unable to share workload of the control circuit board 40, the heating devices in the testing cavity assembly 82 and the heating devices in the sample loading cavity assembly 83 may be directly and electrically connected to the control circuit board 40.

As illustrated in Fig. 10, the testing assembly 90 may include a light generator 91, a light receiver 92 and the testing cavity assembly 82. The light generator 91 is mounted on the support frame 70. The light receiver 92 is mounted on the support frame 70. The testing cavity assembly 82 is mounted on the support base 80, e.g., on the support base main body 81. The testing cavity assembly 82 is the testing cavity assembly 82 of the support base 80, which may be an element shared by both the support base 80 as well as the testing assembly 90, as previously described. The light generator 91 and the light receiver 92 are both electrically connected to the control circuit board 40. The light generator 91 is configured to generate the excitation light, and may generate excitation light under the control of the control circuit board 40. The excitation light may be transmitted to the testing cavity assembly 82 to perform excitation on the test card and generate the fluorescence. The fluorescence may be received by the light receiver 92. The light receiver 92 may generate a testing signal under the control of the control circuit board 40. The testing signal is transmitted to the control circuit board 40, which processes the testing signal to generate diagnostic data.

As illustrated in Fig. 10 and Fig. 15, Fig. 15 is a schematic structural diagram of the light generator 91 in Fig. 10. The number of the light generators 91 may be 2, namely a first light generator 911 and a second light generator 912. Both the first light generator 911 and the second light generator 912 may be fixed to the support frame 70. The excitation light output end of any of the first light generator 911 and the second light generator 912 is coupled with the excitation optical fiber 8212.

As illustrated in Fig. 10, the light receiver 92 is configured to connect to the reception optical fiber 8213 to receive the fluorescence. The testing signal, triggered by the fluorescence, is then transmitted to the control circuit board 40. The light receiver 92 is ring-shaped and arranged around the light generator 91. The light receiver 92 may include an optical sensor, such as a photodiode. The optical sensor, such as a photodiode, may generate an electrical signal when exposed to the fluorescence.

As further illustrated in Fig. 1 and Fig. 2, electronic components, such as a processor and memory etc. may be provided on the control circuit board 40. The control circuit board 40 may control the rotation of the lead screw 12, thereby controlling the sliding position of the test card conveyor base 20 relative to the frame 10. The control circuit board 40 may be electrically connected to the pressure-bearing disc 50, for example, to the first circuit board 56, so as to control the electromagnetic component 52 to attract the tight-pressing member 53 through magnetic force, thereby controlling the first heating component 54 and the second heating component 55 to heat the test card. The control circuit board 40 may control the third driving assembly 64 to drive the card holder 65 to slide relative to the sliding carriage 61. The control circuit board 40 may be electrically connected to the delivery assembly 60, for example, to the third driving assembly 64, so as to control the third driving assembly 64 to drive the card holder 65 for centrifugal treatment of the test card. The control circuit board 40 may be electrically connected to the second circuit board 84, to be indirectly connected with the testing cavity assembly 82, such as the heating devices of the testing cavity assembly 82, thereby controlling the testing base 8211 to heat the test card. The control circuit board 40 may be electrically connected to the second circuit board 84, to be indirectly connected with the sample loading cavity assembly 83, such as the heating devices of the sample loading cavity assembly 83, thereby controlling the sample loading cavity mounting seat 832 to heat the test card. The control circuit board 40 may be electrically connected to the testing assembly 90, such as the light generator 91 of the testing assembly 90, thereby controlling the light generator 91 to emit the excitation light. The control circuit board 40 may further be electrically connected to the testing assembly 90, such as the light receiver 92 of the testing assembly 90, thereby controlling the light receiver 92 to receive the fluorescence. The control circuit board 40 may be electrically connected to the input devices such as the display, the keyboard, and the code scanning device 13, so as to input the controlling instructions into the molecular diagnostic device 100, for example, to the control circuit board 40 of the molecular diagnostic device 100. In this way, the control of the molecular diagnostic device 100 over the test card conveyor base 20 and/or the test card testing base 30 through the control circuit board 40 may be achieved.

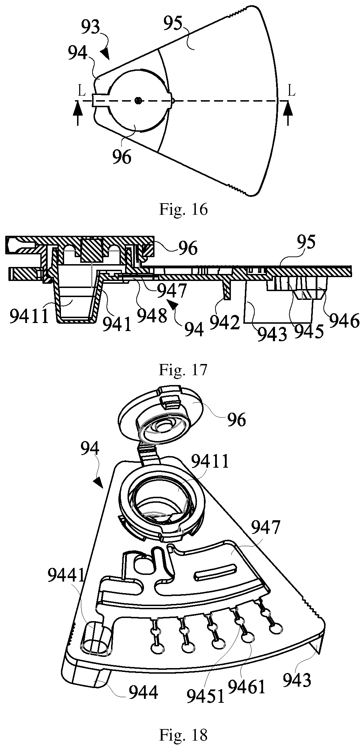

Next, the test card would be described. This test card may be used in the molecular diagnostic device 100 in the above-mentioned embodiments, to complete the test of samples loaded on the test card, and the diagnostic data is formed by a further process. As illustrated in Fig. 16, Fig. 16 is a schematic structural diagram of the test card according to one embodiment of the present disclosure. The test card 93 may also referred to as a molecular diagnostic centrifugal test card or testing card. The test card 93 may include a body 94, an isolation layer 95 and a cover 96. The body 94 is provided with a sample loading cavity, a flow channel, a waste liquid cavity, an isolation cavity, and a testing cavity. The isolation layer 95 covers one side of the body 94. The cover 96 is covered over the sample loading cavity of the body 94.

As illustrated in Fig. 16, Fig. 17 and Fig. 18, Fig. 17 is a cross-sectional view of the test card 93 along line L-L in Fig. 16. Fig. 18 is a schematic three-dimensional structural diagram of the test card 93 in Fig. 16. The body 94 is made of a rigid material, such as plastic etc. The body 94 may have a plate-like structure as a whole. The body 94 is generally fan-shaped or sector-shaped. In some embodiments, the body 94 may be in the shape of a fan ring, a fan blade, or a pie. For example, the body 94 may be formed into a fan shape by connecting two straight edges and an arc-shaped edge sequentially. For example, the body 94 may be formed into a fan ring shape, which is formed by sequentially connecting a straight edge, an outer-side circular arc edge, another straight edge, and an inner-side circular arc edge. Of course, the body 94 may also have other shapes, which will not be detailed here.

In some embodiments, the two straight edges of the fan-shaped structure of the body 94 may form an angle ranging from 40° to 60°. The diameter of the inner circular arc edge may range from 10mm to 100mm. The diameter of the outer circular arc edge may range from 100mm to 200mm. In case the test card with this dimensional structure is adopted, at least 6 test cards 93 may be arranged in a test plane of the molecular diagnostic device 100, thereby forming circular plane, and enabling the at least 6 test cards 93 to be tested simultaneously. In this way, the overall testing efficiency may be enhanced, and a large-scale testing demand may be met.

The body 94 is provided with a mounting portion 941. The mounting portion 941 is positioned near either the center of the outer circular arc edge or the inner circular arc edge of the body 94, and protrudes outward on the side of the body 94 facing away from the isolation layer 95. The body 94 is provided with a sample loading cavity 9411. The sample loading cavity 9411 is recessed on the side near the isolation layer 95, and is positioned opposite to the mounting portion 941. The sample loading cavity 9411 is configured to add samples into itself. The sample loading cavity 9411 is primarily configured for pre-processing of samples (liquid samples). The pre-processing manners of the samples may include one or more of chemical treatment, thermal treatment, enzyme treatment, and physical separation, etc. In some embodiments, the volume of the sample loading cavity 9411 generally ranges from 200 µl to 2000 µl. Dry reagents may be preloaded into the sample loading cavity 9411. The reagent may either be air-dried or heat-dried in situ, or may be added into the sample loading cavity 9411 as freeze-dried reagents.

The body 94 is provided with a second fastening portion 942. The second fastening portion 942 is positioned on the side away from the isolation layer 95 and near the outer circular arc edge. The second fastening portion 942 is configured to engage with the delivery assembly 60, such as the card holder 65 of the delivery assembly 60. In some embodiments, the second fastening portion 942 may be a protrusion or a bump.

The body 94 is provided with a first limiting portion 943 and a second limiting portion 944. The first limiting portion 943 is positioned at the edge of one straight side and near the outer circular arc edge, and is configured to bend outward on the side facing away from the isolation layer 95. The second limiting portion 944 is positioned on another straight side and near the outer circular arc edge, and is configured to protrude outward on the side facing away from the isolation layer 95. The first limiting portion 943 and the second limiting portion 944 of the body 94 cooperate to secure the test card 93 for smooth centrifugal treatment of the test card 93. The body 94 is provided with a waste liquid cavity 9441. The waste liquid cavity 9441 is recessed on the side of the body 94 near the isolation layer 95 and is positioned opposite to the second limiting portion 944. The body 94 is provided with a plurality of connector groups. These plurality of connector groups are evenly distributed along an extension direction of the outer circular arc edge on the side of the body 94 away from the isolation layer 95, and are positioned between the first limiting portion 943 and the second limiting portion 944. Each connector portion group may include a first connector portion 945 and a second connector portion 946. The connection line between the first connector portion 945 and the second connector portion 946 may pass through the center of the outer circular arc edge. The first connector portion 945 is positioned between the mounting portion 941 and the second connector portion 946. The arrangement of the first connector portion 945 and the second connector portion 946 may cooperate with the test card testing base 30, such as an isolation slot 8213 and a testing slot 8212.

The body 94 is provided with an isolation cavity 9451. The isolation cavity 9451 is recessed on the side near the isolation layer 95 and is positioned opposite the first connector portion 945. A meltable isolation body is arranged in the isolation cavity 9451. The isolation body may switch between a melted state and an unmelted state (typically solid). When the test card 93 is not used for testing, the isolation body may be controlled to remain in the unmelted state. In this state, the isolation body may prevent the sample from entering a testing cavity 9461 (as illustrated in Fig. 18) via the flow channel. In some embodiments, the isolation body may be paraffin wax, microcrystalline wax, synthetic wax, or natural wax.

The body 94 is provided with the testing cavity 9461. The testing cavity 9461 is recessed on the side near the isolation layer 95 and is positioned opposite the second connector portion 946. The reagent is arranged in the testing cavity 9461. The isolation cavity 9451 and the testing cavity 9461 are in communication on the side near the isolation layer 95. The isolation body in the isolation cavity 9451 may also be configured to seal and isolate the reagent, preventing the reagent from entering the isolation cavity 9451 in a reverse direction, thereby maintaining the reagent in the testing cavity 9461. During the testing process, the isolation body may be controlled to be in a melted state. At this point, the sample may enter the testing cavity 9461 through the sample loading cavity 9411, where the sample may react with the reagent inside the testing cavity 9461 to complete the testing process.

The reagent and the meltable isolation body are arranged in a stacked configuration within the testing cavity 9461. In some embodiments, the isolation body may be paraffin wax, microcrystalline wax, synthetic wax, or natural wax. The characteristic of the isolation body is that, it is solid at room temperature and low temperatures, and becomes liquid when heated to a specific temperature, and the isolation body does not inhibit the nucleic acid amplification reaction. In some embodiments, the reagent may be a dry reagent. The dry reagent includes one or more of primers and DNA (deoxyribonucleic acid) binding dyes, enzymes, magnesium sulfate, potassium chloride, dNTPs (nucleoside triphosphates) used in the amplification reaction. The dry reagent is loaded into the testing cavity 9461 in the liquid form, and then undergoes a drying process to form the dry reagent. The drying process is conducted at a temperature lower than the melting point of the isolation body. The drying process includes air drying, heating drying, or freeze drying. During the testing and heating process, both the reagent and the isolation body are in liquid form. Since the specific gravity of the isolation body is less than that of the reagent, under an action of a centrifugal force, the isolation body would be displaced from the testing cavity 9461, thereby not affecting the reaction and the testing.

In some embodiments, the isolation body is loaded into the testing cavity 9461 in a melted state and is shaped by natural solidification or cooling. When no testing process is performed, the isolation body may be controlled to remain in the unmelted state, thereby sealing and isolating the reagent for storage. When a testing process is required, the isolation body may be controlled to enter a melted state, such as by heating the test card 93, causing the isolation body to be heated and melt. At this point, under the action of the centrifugal force, the isolation body may be displaced from the testing cavity 9461 and flow into the isolation cavity 9451, allowing the sample to enter the testing cavity 9461. The isolation body would then solidify again to seal or block an inlet of the testing cavity 9461, thereby forming isolated seals between a plurality of testing cavities 9461, allowing for independent reactions or tests within each testing cavity 9461.

The body 94 is provided with a flow channel 947. The flow channel 947 is defined on the side near the isolation layer 95, and is configured to communicate the sample loading cavity 9411 with the isolation cavity 9451.

The body 94 is provided with an abutting slot 948. The abutting slot 948 is positioned on the side away from the isolation layer 95 and is opposite the flow channel 947. The abutting slot 948 is configured to engage with the abutting portion 8324. For example, the abutting portion 8324 may be placed in the abutting slot 948, so as to heat the part of the flow channel 947, preventing moisture from condensing inside the flow channel 947. In this way, an impact of condensation on subsequent testing processes may be reduced, thereby improving the testing accuracy. In some embodiments, the abutting slot 948 may be omitted.

The isolation layer 95 may be a membrane-like structure. Of course, the isolation layer 95 may have other forms of structures. The isolation layer 95 may be made from materials such as sealing adhesives, ultraviolet-curing adhesives, or optical-grade double-sided adhesives. Alternatively, the isolation layer 95 may be made from materials like those of the body 94. The isolation layer 95 may be attached to the body 94. In some embodiments, the isolation layer 95 may for example be sealed and fixed by approaches such as ultrasonic welding, laser welding, or adhesive sealing, so as to isolate the flow channel 947, the waste liquid cavity 9441, the isolation cavity 9451, and the testing cavity 9461. The isolation layer 95 and the body 94 may be integrated as a single-piece structure.

The cover 96 is placed over an opening of the sample loading cavity 9411 to seal and isolate the sample loading cavity 9411. When addition of samples is required, the cover 96 may be opened, and the samples may be added to the sample loading cavity 9411, after which the cover 96 may be closed. The cover 96 may be waterproof and breathable (or, waterproof and permeable), allowing steam generated during the heating process to escape, reducing the internal air pressure of the molecular diagnostic centrifuge test card, thus ensuring excellent ventilation effect. Additionally, the cover 96 may prevent the escape of pollutants such as aerosols and biomolecules generated during the amplification reaction, thereby protecting the personnels and the environment from contamination during the testing process.

When the test card 93 undergoes centrifugal rotation, the sample liquid flows through the sample loading cavity 9411 and the flow channel 947. At this point, the isolation body is solid, which seals the testing cavity 9461, and the sample liquid is unable to flow into the testing cavity 9461. After the sample liquid is heated, the isolation body is heated and melts, and flows into the testing cavity 9461, allowing communication between the flow channel 947 and the testing cavity 9461, thus the sample liquid may flow into the testing cavity 9461. Since the specific gravity of the isolation body is less than that of the reagent, under an action of the centrifugal force, the isolation body would be displaced onto the reagent, thereby not affecting the reaction and the testing, and the testing cavity 9461 may be sealed.

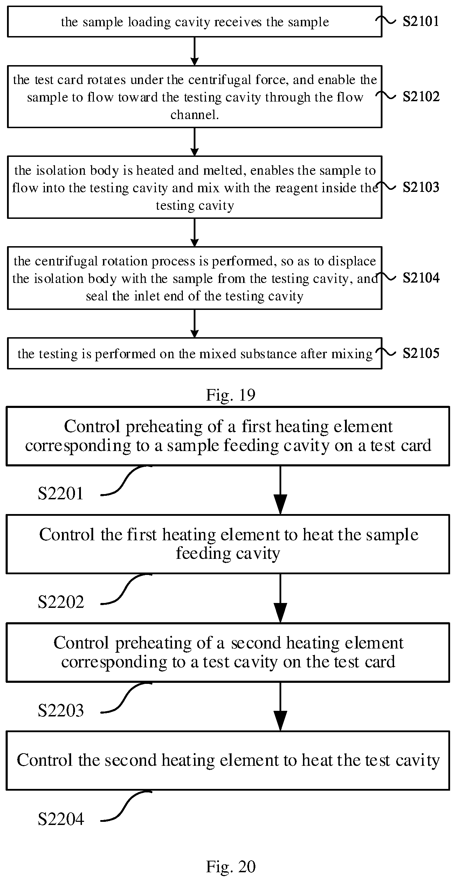

A testing method based on the above-mentioned test card 93 is further provided according to some embodiments of the present disclosure. This method may be applied to the molecular diagnostic device 100 of the above-mentioned embodiments. As illustrated in Fig. 19, Fig. 19 is a flow chart of a usage process of the test card in Fig. 18. The method includes operations at blocks illustrated in Fig. 19.

At block S2101: the sample loading cavity receives the sample.

After the sample is added to the sample loading cavity 9411, the sample needs to undergo heating pre-processing. The heating approaches may include heating through a metal heating block, a heated airflow, or electromagnetic waves (the infrared radiation, the laser, the microwave), etc. The heating region of the test card may be a region in vicinity of the sample loading cavity. The heating process needs to reach a specified temperature, such as 90 °C. Once the specified temperature is reached, the temperature is maintained for 3-10 min according to the specified requirements to achieve the pre-processing. After the pre-processing is completed, the temperature of the sample liquid is reduced to a designated temperature. The designated temperature is for example 60°C.

At block S2102: the test card rotates under the centrifugal force, and enable the sample to flow toward the testing cavity through the flow channel.

The rotation of the test card is controlled to perform centrifugal treatment. For example, the rotation direction may be controlled to be clockwise, the rotation speed greater than 1000 rpm, and the rotation time approximately range from 10-15 seconds. The sample liquid may flow from the sample loading cavity through the flow channel to the testing cavity by rotation, thereby facilitating subsequent filling of the testing cavity with the sample. Any excess sample liquid would enter the waste liquid cavity.

At block S2103: the isolation body is heated and melted, enables the sample to flow into the testing cavity and mix with the reagent inside the testing cavity.

In this process, the region near the testing cavity is required to be heated to a temperature above the melting point of the isolation body, causing the isolation body to melt, such that the sample mixes with the reagent in the testing cavity.

At block S2104: a centrifugal rotation process is performed, so as to displace the isolation body with the sample of the testing cavity, and seal an inlet end of the testing cavity.

The rotation of the test card is again controlled. The rotating direction of the motor is clockwise, the rotating speed is greater than 1000 rpm, and the rotating time ranges from 10-15 seconds. In this process, as the test card rotates, the sample enters the testing cavity, and the isolation body is displaced with the aqueous solution in the testing cavity, thereby moving the isolation body to the inlet end of the testing cavity and completing the sealing of each testing cavity. Then, motor control parameters are adjusted, to make the test card rotate alternately clockwise and counterclockwise. For example, the test card may be controlled to rotate clockwise at 3000 rpm for 1 second, then to rotate counter clockwise at 3000 rpm for 1 second, alternately rotate 10 to 15 times. This alternating clockwise and counterclockwise rotation ensures that, the sample and the reagent are fully dissolved and mixed in the testing cavity.

Meanwhile, after the isolation body is melted by heating, the isolation body flows to the inlet end of the testing cavity under the centrifugal force, thereby sealing the inlet end of the testing cavity.

At block S2105: a testing is performed on the mixed substance after mixing.

In this process, the amplification reaction and the testing are required to be performed. If a real-time testing process is used, the testing and the amplification reaction may be performed simultaneously. If an endpoint testing process is used, the testing is performed after the amplification reaction is completed. The amplification reaction may be carried out by the following approach: heating the region near the testing cavity, with the temperature controlled in the range from 60°C to 75°C; after reaching the specified temperature, the temperature is maintained for 30-60 min as required to complete the amplification reaction.

Next, a control method based on a molecular diagnostic device is described. This control method may be applied to the above-mentioned molecular diagnostic device 100 to perform testing on the above-mentioned test card 93. Specifically, the method may be used to control the heating process of the test card. As illustrated in Fig. 20, Fig. 20 is a flowchart of a control method based on a molecular diagnostic device according to an embodiment of the present disclosure. This method may include operations at blocks illustrated in Fig. 20.

At block S2201: the first heating component for the sample loading cavity on the test card is controlled to perform preheating.

As illustrated in Fig. 16, Fig. 17 and Fig. 18, the test card 93 may include the sample loading cavity 9411 as well as the testing cavity 9461. In this process, the sample loading cavity 9411 of the test card 93 is pre-processed by heating, this facilitates the pre-processing of the sample within the sample loading cavity 9411, which in turn supports subsequent sample testing. In some embodiments, the pre-processing manners of the samples may include one or more of chemical treatment, thermal treatment, enzyme treatment, and physical separation, etc.

In some embodiments, as illustrated in Fig. 3 and Fig. 10, the heating components for heating the sample loading cavity 9411 of the test card 93 may include the second heating component 55 and the fourth heating component 832. The second heating component 55 is configured to heat the test card 93, such as to heat the top of the sample loading cavity 9411 of the test card 93. The fourth heating component 832 is configured to heat the test card 93, such as to heat the bottom of the sample loading cavity 9411. During the heating process performed by the heating components, at least one of the second heating component 55 and the fourth heating component 832 may be configured to heat the sample loading cavity 9411 of the test card 93. Therefore, the heating components, for example the as second heating component 55 and the fourth heating component 832, may be configured to heat the test card 93, such as to heat the sample loading cavity 9411 of the test card 93. In some embodiments, the heating components, such as the second heating component 55 and the fourth heating component 832, may also be arranged in other manners, instead of the arrangement manner illustrated in Figs. 3 and 10.

In some embodiments, as illustrated in Fig. 3 and Fig. 10, the heating components, such as the second heating component 55 and the fourth heating component 832, heat the test card 93, such as the sample loading cavity 9411 of the test card 93, but do not simultaneously heat the test card 93, such as the testing cavity 9461 of the test card 93. This allows the heating components, such as the second heating component 55 and the fourth heating component 832, to be configured more compact. In this way, a case is avoided, in which the heating components, such as the second heating component 55 and the fourth heating component 832, simultaneously heats the test card 93, such as both the sample loading cavity 9411 and the testing cavity 9461, which would require a higher power and would affect the overall power of the molecular diagnostic device 100. The arrangement of the heating components, such as the second heating component 55 and the fourth heating component 832, ensures a safe operation of the molecular diagnostic device 100.

In some embodiments, in the molecular diagnostic device 100 of the above-mentioned embodiments, when the test card 93, such as the sample loading cavity 9411 of the test card 93, is heated, since the heating components, such as the second heating component 55 and the fourth heating component 832, heat the test card 93 from its initial temperature, the testing time on the test card 93 would be caused to extend, thereby lowering the testing efficiency of the molecular diagnostic device 100. Therefore, by using the heating components, such as the second heating component 55 and the fourth heating component 832, to firstly preheat, and then by using the heating components, such as the second heating component 55 and the fourth heating component 832 to heat the test card, such as the sample loading cavity 9411, a significant amount of time may be saved. That is, at least one of the second heating component 55 and the fourth heating component 832 may perform preheating.

In some embodiments, when the heating components, such as the second heating component 55 and the fourth heating component 832, heat the test card 93, such as the sample loading cavity 9411, the heating process may operate in parallel with other operations of the molecular diagnostic device 100. In this way, the time required for heating the test card by the heating components, such as the second heating component 55 and the fourth heating component 832, may be further reduced. The testing efficiency may be enhanced. In some embodiments, during the conveying process of conveying the test card to a heating station, the heating components, such as the second heating component 55 and the fourth heating component 832, are controlled to perform preheat. In some embodiments, the heating station may refer to a position on the test card testing base 30, such as the supporting base 80 of the test card testing base 30, where the test card 93 is placed. In some embodiments, the heating station may be: the sample loading cavity assembly 83 for placing the test card 93, such as the sample loading cavity 9411 of the test card 93; and, the testing cavity assembly 82 for placing the test card 93, such as the testing cavity 9461 of the test card 93. In some embodiments, the conveying process may refer to a process in which the test card 93 is conveyed by the test card conveyor base 20 to the test card testing base 30. In some embodiments, the heating components, such as the second heating component 55 and the fourth heating component 832, may be controlled to preheat to a first temperature. In some embodiments, the conveying process may include at least a delivering process of the test card conveyor base 20 delivering the test card 93. In some embodiments, the conveying process may also include a process of a user setting the testing data. In some embodiments, the conveying process may further include a test card placing process of the user placing the test card 93 onto the card holder 65. The conveying process may include processes prior to heating the test card, which will not be detailed here. In some embodiments, the specific processes involved in the conveying process may vary, leading to different durations for the conveying process. Therefore, the conveying process may be performed simultaneously with the preheating process of the heating components, such as the second heating component 55 and the fourth heating component 832. In some embodiments, the conveying process and the preheating process of the heating components, such as the second heating component 55 and the fourth heating component 832, may either begin sequentially or simultaneously. In some embodiments, the conveying process and the preheating process of the heating components, such as the second heating component 55 and the fourth heating component 832, may either complete sequentially or at the same time. In some embodiments, the preheating process of the heating components, such as the second heating component 55 and the fourth heating component 832, may be completed before the completion of the conveying process. The preheating process of the heating components, such as the second heating component 55 and the fourth heating component 832, may be followed by a temperature maintenance process.

In some embodiments, the first temperature may range from 20°C to 50°C. In some embodiments, the first temperature may be one of 25°C, 30°C, 35°C, 40°C, or 45°C. The first temperature may be adjusted as required.

In some embodiments, the first temperature may be determined based on the test card 93, such as based on the reagent and/or the sample in the sample loading cavity 9411 of the test card 93. That is, the reagents in the sample loading cavity 9411 vary across different test cards 93, leading to different requirements for the first temperature. Therefore, the first temperature may be determined based on the type of test card.

In some embodiments, the first temperature and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the first temperature matching the type of the test card. In some embodiments, the user may input the type of the test card into the molecular diagnostic device 100, the molecular diagnostic device 100 then selects the first temperature matching the type of the test card. Alternatively, the user may directly input the first temperature into the molecular diagnostic device 100.

In some embodiments, as illustrated in Fig. 3, the heating component, such as the second heating component 55, may be one. Therefore, whether one or a plurality of test card(s) 93 is/are placed on the card holder 65, the heating component, such as the second heating component 55, is generally required to perform preheating and subsequent heating.

In some embodiments, as illustrated in Fig. 14, the number and positions of the fourth heating components 832 may be configured to match the number of the test cards 93 on the card holder 65. Therefore, when the sample volume is small, the fourth heating components 832 matching the number and positions of the test cards may be individually controlled, based on the number and positions of the test cards placed on the card holder 65, to perform preheating and subsequent heating, further reducing the overall power consumption of the molecular diagnostic device 100.

At block S2202: the first heating component is controlled to heat the sample loading cavity.

After the heating components, such as the second heating component 55 and the fourth heating component 832, complete the preheating process, the test card 93 is conveyed to the test card testing base 30 via the test card conveyor base 20. At this point, the second heating component 55 and the fourth heating component 832 may clamp the test card 93, such as the sample loading cavity 9411 of the test card 93. The preheated heating components of the second heating component 55 and the fourth heating component 832 may then further heat the test card 93, such as the sample loading cavity 9411, to complete the preheating process of the sample.

In some embodiments, the heating components, such as the second heating component 55 and the fourth heating component 832, may be controlled to heat the sample loading cavity to a second temperature. The first temperature may be less than the second temperature. When the reagent in the sample loading cavity processes the sample, it must be conducted at a specific temperature, such as the second temperature. If the heating temperature has not been reached, the reagent may not process the sample or may process the sample at a slow rate. Therefore, it is necessary to ensure that the sample and reagent in the sample loading cavity remain at the second temperature until the processing of the sample by the reagent has completed.

In some embodiments, the second temperature may range from 40°C to 100°C. In some embodiments, the first temperature may be one of 45°C, 50°C, 55°C, 60°C, 65°C, 70°C, 75°C, 80°C, 90°C, 95°C. The second temperature may be adjusted as required.

In some embodiments, the second temperature may be determined based on the test card 93, such as based on the reagent and/or the sample in the sample loading cavity 9411 of the test card 93. That is, the reagents in the sample loading cavity 9411 vary across different test cards 93, leading to different requirements for the second temperature. Therefore, the second temperature may be determined based on the type of test card.

In some embodiments, the second temperature and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the second temperature matching the type of the test card. In some embodiments, the user may input the type of the test card into the molecular diagnostic device 100, the molecular diagnostic device 100 then selects the second temperature matching the type of the test card. Alternatively, the user may directly input the second temperature into the molecular diagnostic device 100.

In some embodiments, after the heating components, such as the second heating component 55 and the fourth heating component 832, are controlled to heat the sample loading cavity to the second temperature, the heating components, such as the second heating component 55 and the fourth heating component 832, are controlled to maintain the temperature of the sample loading cavity for a first time duration. The temperature maintenance process after the heating process may facilitate the reagent to complete the processing of the sample. In some embodiments, within the first time duration for temperature maintenance, the reagent may complete the processing of the sample, or the processing may be incomplete, but the subsequent testing process may be performed.

In some embodiments, the first time duration may be a fixed value, such as may range from 3 min to 10 min. In some embodiments, the first time duration may be one of the following values: 4 min, 5 min, 6 min, 7 min, 8 min, or 9 min. The first time duration may also be adjusted.

In some embodiments, the first time duration may be determined based on the test card 93 (such as based on the reagent and/or the sample in the sample loading cavity 9411 of the test card 93) and the second temperature. That is, the reagents in the sample loading cavity 9411 vary across different test cards 93, leading to different requirements for the first time duration. Therefore, the first time duration may be determined based on the type of the test card and the second temperature.

In some embodiments, the first time duration and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the first time duration matching the type of the test card. In some embodiments, the user may input the type of the test card into the molecular diagnostic device 100, the molecular diagnostic device 100 then selects the first time duration matching the type of the test card. Alternatively, the user may directly input the first time duration into the molecular diagnostic device 100.

At block S2203: the second heating component for the testing cavity on the test card is controlled to perform preheating.

When the heating components, such as the second heating component 55 and the fourth heating component 832, heat only the test card 93, such as the sample loading cavity 9411 of the test card 93, it is necessary to set up additional heating components to heat the test card 93, such as to heat the testing cavity 9461 of the test card 93. In this way, the heating components responsible for heating the test card 93, such as the testing cavity 9461 of the test card 93, to focus solely on heating the test card 93, such as the testing cavity 9461 of the test card 93, allowing the arrangement more compact. In this way, a case is avoided, in which the heating components, such as the second heating component 55 and the fourth heating component 832, simultaneously heats the test card 93, such as both the sample loading cavity 9411 and the testing cavity 9461, which would require a higher power and would affect the overall power of the molecular diagnostic device 100. The arrangement of the heating components, such as the second heating component 55 and the fourth heating component 832, ensures a safe operation of the molecular diagnostic device 100.

In some embodiments, as illustrated in Fig. 16, Fig. 17 and Fig. 18, heating the testing cavity 9461 of the test card 93 helps to melt the isolation body within the isolation cavity 9451 and the testing cavity 9461, thereby allowing the sample that subject to the centrifugal treatment and the preprocess to enter the testing cavity 9461, where the sample is further processed by the reagent within the testing cavity 9461. In some embodiments, the isolation cavity 9451 of the test card 93 may be omitted. In some embodiments, the meltable isolation body within the isolation cavity 9451 and/or the testing cavity 9461 may be omitted. The sample subject to the centrifugal treatment and the preprocess would enter the testing cavity 9461, and be further processed with the reagent within the testing cavity 9461.

In some embodiments, as illustrated in Fig. 3, Fig. 10 and Fig. 13, the heating components for heating the testing cavity 9461 of the test card 93 may include the first heating component 54 and a third heating component 8211. The first heating component 54 is configured to heat the test card 93, such as to heat the top of the testing cavity 9461 of the test card 93. The third heating component 8211 is configured to heat the test card 93, such as to heat the bottom of the testing cavity 9461. During the heating process performed by the heating components, at least one of the first heating component 54 and the third heating component 8211 may be configured to heat the testing cavity 9461 of the test card 93. Therefore, the heating components, for example the first heating component 54 and the third heating component 8211, may be configured to heat the test card 93, such as to heat the sample loading cavity 9411 of the test card 93. In some embodiments, the heating components, such as the first heating component 54 and the third heating component 8211, may also be arranged in other manners, instead of the arrangement manner illustrated in Figs. 3 and 10, Fig. 13.

In some embodiments, as illustrated in Fig. 3 and Fig. 10, Fig. 13, the heating components, such as the first heating component 54 and the third heating component 8211 heat the test card 93, such as the testing cavity 9461 of the test card 93, but do not simultaneously heat the test card 93, such as the sample loading cavity 9411 of the test card 93. This allows the heating components, such as the first heating component 54 and the third heating component 8211, to be configured more compact. In this way, a case is avoided, in which the heating components, such as the first heating component 54 and the third heating component 8211 simultaneously heat the test card 93, such as heat both the sample loading cavity 9411 and the testing cavity 9461 of the test card 93, which would require a higher power and would affect the overall power of the molecular diagnostic device 100. The arrangement of the heating components, such as the first heating component 54 and the third heating component 8211 ensures a safe operation of the molecular diagnostic device 100.

In some embodiments, as illustrated in Fig. 3, the number, positions of the first heating components 54 may be configured to match the number, positions of the test cards 93 on the card holder 65. Therefore, when the sample volume is small, the first heating components 54 matching the number and positions of the test cards may be individually controlled, based on the number and positions of the test cards placed on the card holder 65, to perform preheating and subsequent heating, further reducing the overall power consumption of the molecular diagnostic device 100.

In some embodiments, as illustrated in Fig. 10 and Fig. 13, the number, positions of the third heating components 8211 may be configured to match the number, positions of the test cards 93 on the card holder 65. Therefore, when the sample volume is small, the third heating components 8211 matching the number and positions of the test cards may be individually controlled, based on the number and positions of the test cards placed on the card holder 65, to perform preheating and subsequent heating, further reducing the overall power consumption of the molecular diagnostic device 100.

In some embodiments, in the molecular diagnostic device 100 of the above-mentioned embodiments, when the test card 93, such as the testing cavity 9461 of the test card 93, is heated, since the heating components, such as the first heating component 54 and the third heating component 8211 heat the test card 93 from its initial temperature, the testing time on the test card 93 would be caused to extend, thereby lowering the testing efficiency of the molecular diagnostic device 100. Therefore, by using the heating components, such as the first heating component 54 and the third heating component 8211 to firstly preheat, and then by using the heating components, such as the first heating component 54 and the third heating component 8211 to heat the test card, such as the testing cavity 9461 of the test card, a significant amount of time may be saved. That is, at least one of the first heating component 54 and the third heating component 8211 may perform preheating.

In some embodiments, when the heating components, such as the first heating component 54 and the third heating component 8211 heat the test card 93, such as the testing cavity 9461 of the test card 93, the heating process may operate in parallel with other operations of the molecular diagnostic device 100. In this way, the time required for heating the test card by the heating components, such as the first heating component 54 and the third heating component 8211 may be further reduced. The testing efficiency may be enhanced. In some embodiments, the heating components, such as the first heating component 54 and the third heating component 8211, are controlled to complete the preheating process before the centrifugal process is completed. In some embodiments, the heating components, such as the first heating component 54 and the third heating component 8211, is controlled to complete the preheating process when the centrifugal process is completed. In some embodiments, after the heating components, such as the second heating component 55 and the fourth heating component 832, only heat the test card 93, such as the sample loading cavity 9411 of the test card 93, the test card 93 is required to undergo centrifugal treatment. This ensures that the processed sample is conveyed to the location of the isolation cavity 9451, so that the processed sample may be processed and tested in the testing cavity 9461. In some embodiments, the heating components, such as the first heating component 54 and the third heating component 8211 may be controlled to preheat to a third temperature. In some embodiments, the centrifugal process and the preheating process of the heating components, such as the first heating component 54 and the third heating component 8211 may either begin sequentially or simultaneously. In some embodiments, the conveying process and the preheating process of the heating components, such as the first heating component 54 and the third heating component 8211 may either complete sequentially or at the same time. In some embodiments, the preheating process of the heating components, such as the first heating component 54 and the third heating component 8211 may be completed before the completion of the conveying process. The preheating process of the heating components, such as the first heating component 54 and the third heating component 8211 may be followed by a temperature maintenance process.

In some embodiments, the third temperature may range from 40°C to 70°C. In some embodiments, the first temperature may be one of 45°C, 50°C, 55°C, 60°C, or 65°C. The third temperature may be adjusted as required.

In some embodiments, the third temperature may be determined based on the isolation body in the test card 93, such as in the isolation cavity 9451 or the testing cavity 9461. In other words, since the isolation body differs in various test cards 93, the requirement for the third temperature also varies. Therefore, the third temperature may be determined based on the type of test card.

In some embodiments, the third temperature may be determined based on the reagent in the test card 93, such as in the testing cavity 9461 of the test card 93. In other words, since the reagent differs in various test cards 93, the requirement for the third temperature also varies. Therefore, the third temperature may be determined based on the type of test card.

In some embodiments, the third temperature and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the third temperature matching the type of the test card. In some embodiments, the user may input the type of the test card into the molecular diagnostic device 100, the molecular diagnostic device 100 then selects the third temperature matching the type of the test card. Alternatively, the user may directly input the third temperature into the molecular diagnostic device 100.

At block S2202: the second heating component is controlled to heat the testing cavity.

After the heating components, such as the first heating component 54 and the third heating component 8211 complete the preheating process, the test card 93 also complete the centrifugal treatment and is conveyed to the test card testing base 30 via the test card conveyor base 20. At this point, the first heating component 54 and the third heating component 8211 may clamp the test card 93, such as the testing cavity 9461 of the test card 93. The preheated heating components of the first heating component 54 and the third heating component 8211 may then further heat the test card 93, such as the testing cavity 9461 of the test card 93, to complete the preheating process of the sample.

In some embodiments, the heating components, such as the first heating component 54 and the third heating component 8211 may be controlled to heat the testing cavity to a fourth temperature. The third temperature may be less than the fourth temperature. In some embodiments, the isolation body in the test card 93, such as in the isolation cavity 9451 or the testing cavity 9461, must melt at a specific temperature, such as at the fourth temperature. The isolation body would not melt if the heating temperature does not reach the specific temperature, such as the fourth temperature. Therefore, the isolation body in the sample loading cavity must be controlled to reach the fourth temperature until it melts. In some embodiments, after the isolation body melts, further centrifugal treatment and heating process are performed to enable the processed sample in the testing cavity 9461 to be further treated by the reagent in the testing cavity 9461. For example, the temperature may be maintained at the fourth temperature, or the temperature may be further increased, until the treatment of the sample by the reagent is completed. In some embodiments, when no isolation body is present in the test card 93, the sample subject to the centrifugal treatment would be located in the testing cavity 9461. By heating the testing cavity 9461 to the fourth temperature, the processed sample may be further treated by the reagent in the testing cavity 9461 until the treatment of the sample by the reagent has completed.

In some embodiments, the fourth temperature may range from 60°C to 100°C. In some embodiments, the fourth temperature may be one of 65°C, 70°C, 75°C, 80°C, 90°C, 95°C. The fourth temperature may be adjusted as required.

In some embodiments, the fourth temperature may be determined based on the isolation body in the test card 93, such as in the isolation cavity 9451 or the testing cavity 9461. In other words, since the isolation body differs in various test cards 93, the requirement for the fourth temperature also varies. Therefore, the fourth temperature may be determined based on the type of test card.

In some embodiments, the fourth temperature may be determined based on the reagent in the test card 93, such as in the testing cavity 9461 of the test card 93. In other words, since the reagent differs in various test cards 93, the requirement for the fourth temperature also varies. Therefore, the fourth temperature may be determined based on the type of test card.

In some embodiments, the fourth temperature and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the fourth temperature matching the type of the test card. In some embodiments, the user may input the type of the test card into the molecular diagnostic device 100, the molecular diagnostic device 100 then selects the fourth temperature matching the type of the test card. Alternatively, the user may directly input the fourth temperature into the molecular diagnostic device 100.

In some embodiments, after the heating components, such as the first heating component 54 and the third heating component 8211 are controlled to heat the testing cavity to the fourth temperature, the heating components, such as the first heating component 54 and the third heating component 8211 are controlled to maintain the temperature of the testing cavity for a second time duration. In some embodiments, during the temperature maintenance process following the heating process, the isolation body in the test card 93, such as in the isolation cavity 9451 or the testing cavity 9461 of the test card 93 may be melted. In some embodiments, during the temperature maintenance process after the heating process, the isolation body in the test card 93, such as in the isolation cavity 9451 or the testing cavity 9461 of the test card 93, may be melted, while simultaneously further processing of the processed sample by the reagent in the test card 93, such as in the testing cavity 9461 of the test card 93 may be facilitated. In some embodiments, during the temperature maintenance process following the heating process, further processing of the processed sample by the reagent in the test card 93, such as in the testing cavity 9461 of the test card 93 may be facilitated.

In some embodiments, during the second time duration for temperature maintenance, only the isolation body in test card 93, such as in the isolation cavity 9451 or the testing cavity 9461 of the test card 93 is melted.

In some embodiments, during the second time duration for temperature maintenance, only the processed sample is further processed by the reagent in the test card 93, such as in the testing cavity 9461 of the test card 93.

In some embodiments, during the second time duration for temperature maintenance, the isolation body in the test card 93, such as in the isolation cavity 9451 or the testing cavity 9461 of the test card 93, is melted, and the processed sample is further processed by the reagent in the test card 93, such as in the testing cavity 9461 of the test card 93. In some embodiments, the second duration solely refers to a time duration during which the processed sample is further processed by the reagent in the test card 93, such as in the testing cavity 9461 of the test card 93.

In some embodiments, the second time duration may be a fixed value, such as may range from 30 min to 60 min. In some embodiments, the second time duration may be one of 35min, 40min, 45min, 50min, 55min. The second time duration may also be adjusted.

In some embodiments, the second time duration may be determined based on the isolation body in the test card 93 (such as in the isolation cavity 9451 or the testing cavity 9461 of the test card 93) and/or the fourth temperature. In other words, since the isolation body differs in various test cards 93, the requirement for the second time duration also varies. Therefore, the second time duration may be determined based on the type of test card.

In some embodiments, the second time duration may be determined based on the reagent in the test card 93 (such as in the testing cavity 9461 of the test card 93), the fourth temperature. In other words, since the reagent differs in various test cards 93, the requirement for the second time duration also varies. Therefore, the second time duration may be determined based on the type of the test card and the fourth temperature.

In some embodiments, the second time duration and the type of the test card may be preset in the molecular diagnostic device 100. The molecular diagnostic device 100 may use the code scanning device 13 to perform image scanning of the information identifier on the card holder 65, thereby determine the type of the test card 93, then the molecular diagnostic device 100 selects the second time duration matching the type of the test card. In some embodiments, the user may input the type of the test card into the molecular diagnostic device 100, the molecular diagnostic device 100 then selects the second time duration matching the type of the test card. Alternatively, the user may directly input the second time duration into the molecular diagnostic device 100.

The terms "first temperature," "second temperature," "third temperature," "fourth temperature," and "temperature" etc. may be interchangeable in some embodiments. For example, in some embodiments, the "first temperature" in other embodiments may be referred to as the "second temperature," and correspondingly, the "second temperature" in the other embodiments may be referred to as the "first temperature."

Similarly, the terms "first time duration," "second time duration," and "time duration" may be interchangeable in some embodiments. For example, in some embodiments, the "first time duration" in other embodiments may be referred to as the "second time duration", and correspondingly, the "second time duration" in the other embodiments may be referred to as the "first time duration".

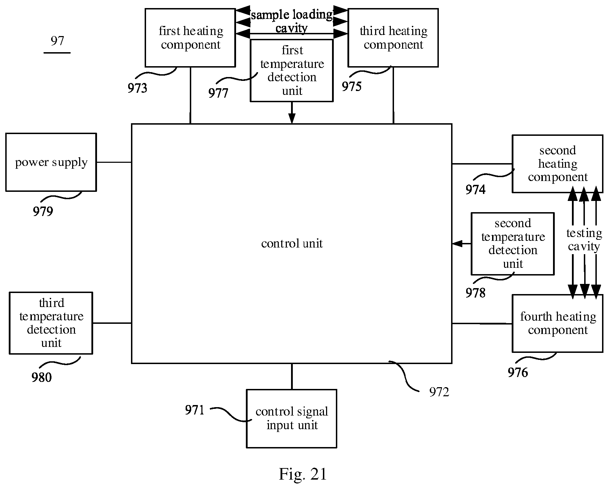

A molecular diagnostic device will be described hereinafter. The molecular diagnostic device may be applied in the above-mentioned method. As illustrated in Fig. 21, Fig. 21 is a structural diagram of the molecular diagnostic device according to one embodiment of the present disclosure. The molecular diagnostic device 97 may include a first heating component 973, a second heating component 974 and a control unit 972. The first heating component 973 is configured for the sample loading cavity of the test card. The second heating component 974 is configured for the testing cavity of the test card. The control unit 972 is connected to the first heating component 973 and the second heating component 974 respectively, and is configured to control the first heating component 973 to perform preheating, heat the sample loading cavity after preheating, and then control the second heating component 974 to perform preheating and heat the testing cavity after preheating.

In some embodiments, the first heating component 973 may be one of the second heating component 55 and the fourth heating component 832 of the molecular diagnostic device 100 in the above-mentioned embodiments.

In some embodiments, the second heating component 974 may be one of the first heating component 54 and the third heating component 8211 of the molecular diagnostic device 100 in the above-mentioned embodiments.

In some embodiments, the control unit 972 may be the processor of the control circuit board 40 of the molecular diagnostic device 100 in the above-mentioned embodiments.

In some embodiments, the control unit 972 is configured to control the first heating component 973 in the heating station to perform preheating during the conveying process of conveying the test card to the heating station.

In some embodiments, the control unit 972 controls the first heating component 973 to complete preheating before the completion of the conveying process, and to maintain the temperature after the preheating process is completed. In some embodiments, the control unit 972 controls the first heating component 973 to complete preheating at the end of the conveying process.

In some embodiments, the main control unit is configured to control the first heating component 973 to preheat to the first temperature, and to control the first heating component 973 to heat the sample loading cavity to the second temperature. The first temperature is less than the second temperature.

In some embodiments, the main control unit 972 is configured to obtain the first temperature matching the type of the test card. In some embodiments, the molecular diagnostic device 97 further includes a control signal input unit 971. The control signal input unit 971 is configured to input the first temperature, or the control signal input unit 971 is configured to input the type of the test card.

In some embodiments, the control signal input unit 971 may be an input device, such as a code scanning device 13, a mouse, a keyboard, a display etc.

In some embodiments, the main control unit 972 is configured to control the first heating component 973 to maintain the temperature of the sample loading cavity for the first time duration.

In some embodiments, the main control unit 972 is configured to obtain the second temperature and the first time duration matching the type of the test card. In some embodiments, the control signal input unit 971 is configured to input the first time duration and the second temperature.

In some embodiments, the main control unit 972 is configured to control the second heating component 974 to perform preheating during the centrifugal process of the test card. In some embodiments, the main control unit 972 is configured to control the second heating component 974 to complete preheating before the completion of the centrifugal process, and to maintain the temperature after the preheating process is completed. In some embodiments, the main control unit 972 is configured to control the second heating component 974 to complete preheating at the end of the centrifugal process.

In some embodiments, the main control unit 972 is configured to control the second heating component 974 to preheat to the third temperature, and to control the second heating component 974 to heat the testing cavity to the fourth temperature. The third temperature is less than the fourth temperature.

In some embodiments, the main control unit 972 is configured to obtain the third temperature matching the type of the test card. In some embodiments, the control signal input unit 971 is configured to input the third temperature.

In some embodiments, the main control unit 972 is configured to obtain the fourth temperature and the second time duration matching the type of the test card. In some embodiments, the control signal input unit 971 is configured to input the fourth temperature and the second time duration.

In some embodiments, the main control unit 972 is configured to control the first heating component 973 and the third heating component 975 to heat the sample loading cavity. In some embodiments, the first heating component 973 may be one of the second heating component 55 and the fourth heating component 832 of the molecular diagnostic device 100 in the above-mentioned embodiments. the third heating component 975 may be one of the second heating component 55 and the fourth heating component 832 of the molecular diagnostic device 100 in the above-mentioned embodiments.

In some embodiments, the main control unit 972 is configured to control the second heating component 974 and the fourth heating component 976 to heat the testing cavity.

In some embodiments, the second heating component 974 may be one of the first heating component 54 and the third heating component 8211 of the molecular diagnostic device 100 in the above-mentioned embodiments. the fourth heating component 976 may be another one of the first heating component 54 and the third heating component 8211 of the molecular diagnostic device 100 in the above-mentioned embodiments.

In some embodiments, the molecular diagnostic device 97 may include a first temperature detection unit 977. The first temperature detection unit 977 is configured to detect the temperature of the sample loading cavity between the first heating component 973 and/or the third heating component 975.

In some embodiments, the main control unit 972 receives the temperature data detected by the first temperature detection unit 977, and controls the first heating component 973 and/or the third heating component 975 to heat the test card based on the temperature data.

In some embodiments, the first temperature detection unit 977 may be mounted on the fourth heating component 832 and/or the second heating component 55 in the molecular diagnostic device 100.

In some embodiments, the molecular diagnostic device 97 may include a second temperature detection unit 978. The second temperature detection unit 978 is configured to detect the temperature of the testing cavity between the second heating component 974 and/or the fourth heating component 976.

In some embodiments, the main control unit 972 receives the temperature data detected by the second temperature detection unit 978, and controls the second heating component 974 and/or the fourth heating component 976 to heat the test card based on the temperature data.

In some embodiments, the second temperature detection unit 978 may be mounted on the third heating component 8211 and/or the first heating component 54 in the molecular diagnostic device 100.

In some embodiments, the molecular diagnostic device 97 may include a power supply 971. The power supply 971 may provide electrical energy for a normal operation of the molecular diagnostic device 97.

In some embodiments, the molecular diagnostic device 97 may include a third temperature detection unit 980. The third temperature detection unit 980 may detect the temperature inside the molecular diagnostic device 97 and may be configured to detect the temperature both inside and outside the molecular diagnostic device 97. The main control unit 972 receives the temperature data detected by the third temperature detection unit 980, and controls at least one of the first heating component 973, the third heating component 975, the second heating component 974, and the fourth heating component 976 to heat the test card based on the temperature data. Of course, the main control unit 972 may also stop heating the test card or halt the operation of the molecular diagnostic device 97 based on the temperature data.

In some embodiments, the third temperature detection unit 980 may be mounted on the frame 10 in the molecular diagnostic device 100. In the several embodiments provided in the present disclosure, the disclosed methods and devices may be implemented in other ways. For example, the device embodiments described above are merely illustrative. For example, the division of modules or units is only a logical function division, and there may be other division manners in actual embodiments. For example, multiple units or components may be combined or integrated into another system. Or some features may be ignored or not implemented.

The units illustrated as separate components may or may not be physically separate, and the components illustrated as units may or may not be physical units. The units may be located in one place or may be distributed on multiple network units. Some or all the units may be selected as per actual needs to fulfill the object of the present disclosure.

In addition, each functional unit in embodiments of the present disclosure may be integrated into one processing unit, or may be physically separate units, or two or more units may be integrated into one unit. The above-mentioned integrated units may be embodied in the form of hardware or software functional unit.

The above are only implementations of the present disclosure, and do not limit the patent scope of the present disclosure. Any equivalent changes to the structure or processes made by the description and drawings of this application or directly or indirectly used in other related technical field are included in the protection scope of this application.

## Claims

1. A control method based on a molecular diagnostic device, comprising:
controlling a first heating component for a sample loading cavity of a test card to perform preheating;
controlling the first heating component to heat the sample loading cavity;
controlling a second heating component for a testing cavity of the test card to perform preheating; and
controlling the second heating component to heat the testing cavity.

2. The method as claimed in claim 1, wherein
the controlling the first heating component for the sample loading cavity of the test card to perform preheating comprises:
controlling the first heating component in a heating station to perform preheating during a conveying process of conveying the test card to the heating station.

3. The method as claimed in claim 2, wherein
the controlling the first heating component in the heating station to perform preheating during the conveying process of conveying the test card to the heating station comprises:
controlling the first heating component to complete the preheating before completion of the conveying process, and maintaining a temperature after the preheating is completed; or
controlling the first heating component to complete the preheating at an end of the conveying process.

4. The method as claimed in claim 1, wherein
the controlling the first heating component for the sample loading cavity of the test card to perform preheating comprises:
controlling the first heating component to preheat to a first temperature;
the controlling the first heating component to heat the sample loading cavity comprises:
controlling the first heating component to heat the sample loading cavity to a second temperature;
wherein the first temperature is less than the second temperature.

5. The method as claimed in claim 4, wherein
before controlling the first heating component to preheat to the first temperature, the method further comprises:
obtaining the first temperature matching a type of the test card.

6. The method as claimed in claim 4, wherein
after the controlling the first heating component to heat the sample loading cavity to the second temperature, the method further comprises:
controlling the first heating component to maintain a temperature of the sample loading cavity for a first time duration.

7. The method as claimed in claim 6, wherein
before the controlling the first heating component to heat the sample loading cavity to the second temperature, the method further comprises:
obtaining the second temperature and the first time duration matching a type of the test card.

8. The method as claimed in claim 1, wherein
the controlling the second heating component for the testing cavity of the test card to perform preheating comprises:
controlling the second heating component to perform preheating during a centrifugal process of the test card.

9. The method as claimed in claim 8, wherein
the controlling the second heating component to perform preheating during the centrifugal process of the test card comprises:
controlling the second heating component to complete the preheating before completion of the centrifugal process, and maintaining a temperature after the preheating is completed; or
controlling the second heating component to complete the preheating at an end of the centrifugal process.

10. The method as claimed in claim 1, wherein
the controlling the second heating component for the testing cavity of the test card to perform preheating comprises:
controlling the second heating component to preheat to a third temperature;
the controlling the second heating component to heat the testing cavity comprises:
controlling the second heating component to heat the testing cavity to a fourth temperature;
wherein the third temperature is less than the fourth temperature.

11. The method as claimed in claim 10, wherein
before controlling the second heating component to preheat to the third temperature, the method further comprises:
obtaining the third temperature matching a type of the test card.

12. The method as claimed in claim 10, wherein
after the controlling the second heating component to heat the testing cavity to the fourth temperature, the method further comprises:
controlling the second heating component to maintain a temperature of the testing cavity for a second time duration.

13. The method as claimed in claim 12, wherein
before the controlling the second heating component to heat the testing cavity to the fourth temperature, the method further comprises:
obtaining both the fourth temperature and the second time duration matching a type of the test card.

14. The method as claimed in claim 1, wherein
the controlling the first heating component for the sample loading cavity of the test card to perform preheating comprises:
controlling both the first heating component and a third heating component for the sample loading cavity to perform preheating;
wherein the first heating component and the third heating component are configured to clamp the sample loading cavity;
the controlling the first heating component to heat the sample loading cavity comprises:
controlling the first heating component and the third heating component to heat the sample loading cavity.

15. The method as claimed in claim 1, wherein
the controlling the second heating component for the testing cavity of the test card to perform preheating comprises:
controlling both the second heating component and a fourth heating component for the testing cavity to perform preheating;
wherein the second heating component and the fourth heating component are configured to clamp the testing cavity; and
wherein the controlling the second heating component to heat the testing cavity comprises:
controlling both the second heating component and the fourth heating component to heat the testing cavity.

16. A molecular diagnostic device, comprising:
a first heating component, configured to be arranged matching a sample loading cavity of a test card;
a second heating component, configured to be arranged matching a testing cavity of the test card; and
a control unit:
connected to the first heating component and the second heating component respectively;
configured to control the first heating component to perform preheating, heat the sample loading cavity after the preheating; and
configured to control the second heating component to perform preheating and heat the testing cavity after the preheating.

17. The molecular diagnostic device as claimed in claim 16, wherein
the control unit is configured to control the first heating component in a heating station to perform the preheating during a conveying process of conveying the test card to the heating station.

18. The molecular diagnostic device as claimed in claim 17, wherein
the control unit is configured to:
control the first heating component to complete preheating before completion of the conveying process, and maintain a temperature after the preheating is completed; or
control the first heating component to complete the preheating at an end of the conveying process.

19. The molecular diagnostic device as claimed in claim 16, wherein
the control unit is configured to control the first heating component to preheat to a first temperature; and
the control unit is configured to control the first heating component to heat the sample loading cavity to a second temperature;
wherein the first temperature is less than the second temperature.

20. The molecular diagnostic device as claimed in claim 19, wherein
before the control unit controlling the first heating component to preheat to the first temperature, the control unit is configured to obtain the first temperature matching a type of the test card.
